(19) Europäisches Patentamt
European Patent Office
Office européen des brevets

(11) Veröffentlichungsnummer: **0 261 380 B1**

(12) # EUROPÄISCHE PATENTSCHRIFT

(45) Veröffentlichungstag der Patentschrift: **03.10.90**

(51) Int. Cl.[5]: **C07D 213/20**, H01B 1/12, C09D 5/24

(21) Anmeldenummer: **87111793.3**

(22) Anmeldetag: **14.08.87**

(54) **Schmelzbare, elektrisch leitfähige TCNQ-Komplexe, Verfahren zu ihrer Herstellung und ihre Verwendung.**

(30) Priorität: **26.08.86 DE 3628905**

(43) Veröffentlichungstag der Anmeldung: **30.03.88 Patentblatt 88/13**

(45) Bekanntmachung des Hinweises auf die Patenterteilung: **03.10.90 Patentblatt 90/40**

(84) Benannte Vertragsstaaten: **BE CH DE FR GB IT LI NL**

(56) Entgegenhaltungen:
**EP-A- 0 160 915**
**EP-A- 0 161 987**
**DE-A- 2 329 492**
**DE-B- 2 826 485**

**PATENT ABSTRACTS OF JAPAN, Band 8, Nr. 126 (C-228)[1563], 13. Juni 1984; & JP - A - 59 36662 (KOGYO GIJUTSUIN) 28.02.1984**
**JOURNAL OF THE AMERICAN CHEMICAL SOCIETY, Band 84, Nr. 17, September 1962, Seiten 3374-3387; L.R. MELBY et al.: "Substituted Quinodimethans. II. Anion-radical Derivatives and Complexes of 7,7,8,8-Tetracyanoquinodimethan"**
**CHEMICAL ABSTRACTS, Band 85, Nr. 9, 30. August 1976, Seite 515, Abstrakt Nr. 62479b, Columbus, Ohio, US; V. KAMPARS et al.: "Characteristics of**

(73) Patentinhaber: **BAYER AG, D-5090 Leverkusen 1 Bayerwerk(DE)**

(72) Erfinder: **Kossmehl, Gerhard, Prof. Dr., Grabenstrasse 38f, D-1000 Berlin 45(DE)**
Erfinder: **Kabbeck-Kupijai, Detlef, Setheweg 12, D-1000 Berlin 22(DE)**
Erfinder: **Jonas, Friedrich, Dr., Krugenofen 15, D-5100 Aachen(DE)**

(56) Entgegenhaltungen: (Fortsetzung)
**pi-electron structure of betainelike active methylene group derivatives. VI. Character of long-wave absorption bands and charge transfer complexes of 1,3-indandione onium betaines", LATV. PSR. ZINAT. AKAD. VESTIS, KIM. SER. 1975 (6), 727-733 in Verbindung mit CHEMICAL ABSTRACTS, Band 85, Formula Index, Juli-Dezember 1976, Teil A-c15, Seite 1025F, rechte Spalte, Zeilen 52-56, sowie Teil C16-Z, Seite 1608F, linke Spalte, Zeilen 55,58-61**
**ZEITSCHRIFT FÜR CHEMIE, Band 10, Nr. 12, 1970, Seite 463; B. LIPKE: "Zur Kenntnis von N-(Phenyl)-pyridiniumsalzen**
**Chemistry Letters, pp 709-712, 1986**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 261 380 B1

ACTORUM AG

## Beschreibung

Die Erfindung betrifft neue schmelzbare, elektrisch leitfähige Komplexsalze des 7,7,8,8-Tetracyanop-chinodimethans (TCNQ).

Komplexsalze des TCNQ und ihre Verwendung als elektrisch leitfähige Verbindungen sind bekannt (siehe z.B. J. Am. Chem. Soc. Vol. 84 (1962), Seiten 3374–3387; die US-PS 3 941 792 und 4 580 855, die deutsche Offenlegungsschrift 3 417 466 und die EP-A 161 987). Diese Komplexsalze entsprechen der allgemeinen Formel

$$K^{n\oplus}\ (TCNQ^{\ominus\cdot})_n\ (TCNQ)_m \qquad (I)$$

in der
$K^{n+}$ ein Kation und
n eine ganze, der Wertigkeit von K entsprechende Zahl ist, und
m die Anzahl der Mole an neutralem TCNQ angibt, die in einem Mol des Komplexsalzes enthalten ist; für m gilt:

$0 \leq m \leq 2\,n$ vorzugsweise $0 \leq m \leq n$.

Als Kationen $K^{n\oplus}$ wurden bislang Alkali- und Erdalkalimetallionen, ferner Arsonium-, Phosphonium- und Ammoniumionen vorgeschlagen. Als Ammoniumionen wurden vor allem solche verwendet, die sich von tertiären Aminen, wie Trialkylaminen, Dialkylanilinen und Pyridinen, und quartären Ammoniumverbindungen, wie Chinolinium-, Isochinolinium- und Pyridinium-Verbindungen oder aber deren N-Alkyl-Derivaten ableiten.

Von den bislang bekannten TCNQ-Komplexen sind die Salze der Formel (I), in denen $K^{n\oplus}$ für ein Ammoniumion, z.B. ein (N-Alkyl-)pyridinium-, (N-Alkyl-)chinolinium-, (N-Alkyl-)isochinoliniumion oder ein Phosphoniumion steht, schmelzbar. Diese schmelzbaren Komplexe haben aber den Nachteil, daß ihre elektrische Leitfähigkeit beim Schmelzen um mehrere Zehnerpotenzen abnimmt (N-Alkylpyridinium-TCNQ-Komplexe wie der N-Methyl-pyridinium TCNQ-Komplex und die in der EP-A 161 987 beschriebenen amphiphilen $N$–$C_{12}$–$C_{30}$-Alkyl-pyridinium-jodid-Komplexsalze) oder daß sie in der Schmelze zwar kurze Zeit stabil sind, aber dafür eine für viele Anwendungsgebiete nicht ausreichende elektrische Leitfähigkeit aufweisen (Isochinolinium- und $N$–$C_{12}$–$C_{30}$-Alkyl-pyridinium-TCNQ-Komplexsalze).

Überraschenderweise wurde nun gefunden, daß TCNQ-Komplexe der Formel (I), in der $K^{n\oplus}$ für ein N-Phenyl pyridinium-Ion steht, nicht nur ein günstiges Schmelzverhalten und eine hohe thermische Stabilität, sondern gleichzeitig auch eine hervorragende elektrische Leitfähigkeit aufweisen.

Die Erfindung betrifft daher TCNQ-Komplexe der Formel

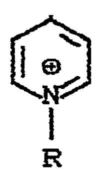 $(TCNQ^{\ominus\cdot})(TCNQ)_m$ (II)

in der
m Null oder eine ganze oder gebrochene Zahl von 0 bis 2, vorzugsweise von 0 bis 1, ist und
R für einen gegebenenfalls durch $C_1$–$C_4$-Alkyl-, $C_1$–$C_4$-Alkoxy-, $C_1$–$C_5$-Acyl-Reste und/oder Halogenatome substituierten Phenylrest steht.

Die erfindungsgemäßen TCNQ-Komplexe der Formel (II) können nach an sich bekannten Verfahren, z.B, den in J. Am. Chem. Soc. 84, (1962), Seiten 3374 ff, insbesondere auf Seite 3380 beschriebenen Verfahren hergestellt werden. Eines dieser Verfahren verläuft nach folgender Reaktionsgleichung

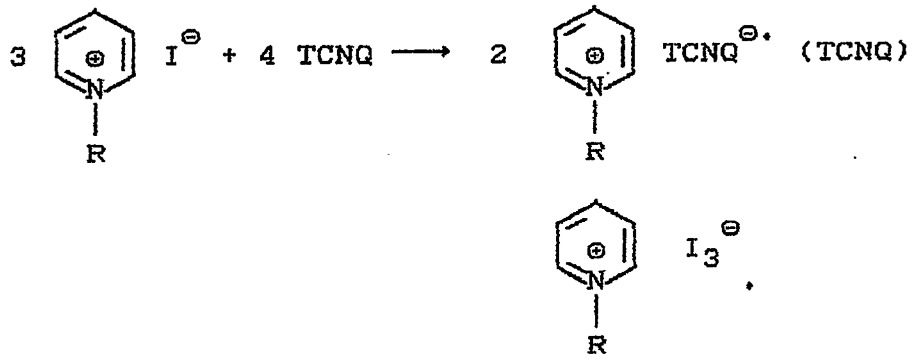

Die Umsetzung der N-Phenyl-pyridiniumiodide mit TCNQ wird vorzugsweise in organischen Lösungsmitteln bei Temperaturen unterhalb von 150°C vorzugsweise bei Temperaturen von 20 bis 100°C vorgenommen.

Geeignete Lösungsmittel für diese Umsetzung sind z.B. Halogenkohlenwasserstoffe, z.B. Methylenchlorid, Chloroform, Tetrachlormethan, 1,2-Dichlorethan, 1,1,2-Trichlorethan; Acetonitril; Alkohole, z.B. Methanol, Ethanol, Isopropanol; alphatische Ketone, z.B. Aceton, Methylethylketon; acyclische oder cyclische Ether, z.B. Diethylether, Tetrahydrofuran. Die Reaktionspartner, TCNQ und N-Aryl-pyridiniumiode, werden in einem Molverhältnis von 1:0,5–1,5 eingesetzt.

Ein anderes Verfahren verläuft nach folgender Reaktionsgleichung (X = Anion, vorzugsweise Halogenidion; M = Metallion, vorzugsweise $Li^{\oplus}$):

Stufe 1:

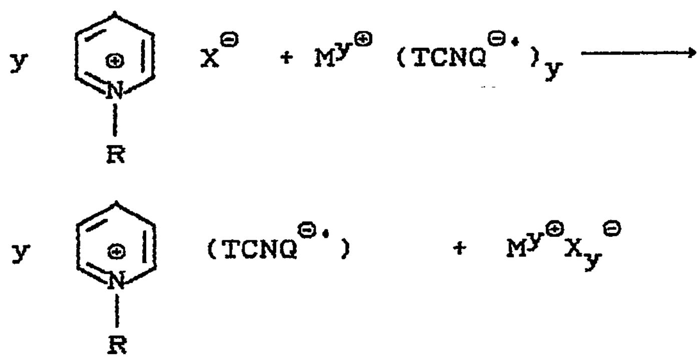

Stufe 2:

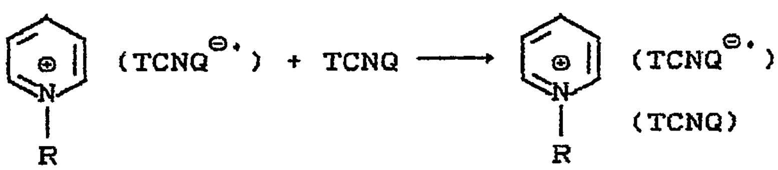

In Stufe 1 wird die Umsetzung vorzugsweise in wäßrigen Alkoholen, in Stufe 2 vorzugsweise in Acetonitril vorgenommen.

Wegen ihres günstigen Schmelzverhaltens und ihrer hohen thermischen Stabilität eignen sich die erfindungsgemäßen N-Phenyl-pyridinium-TCNQ-Komplexe der Formel (II) besonders zur Herstellung elektrisch leitender Überzüge auf Substraten durch Aufschmelzen.

Die Erfindung betrifft daher auch die Verwendung der erfindungsgemäßen N-Phenyl-pyridinium-TCNQ-Komplexe zur Herstellung elektrisch leitfähiger Überzüge durch Aufschmelzen auf Substraten oder aber durch Tauchbeschichtung von Substraten.

Die erfindungsgemäßen TCNQ-Komplexe lassen sich auch aus der Schmelze zu leitenden Überzügen verarbeiten. Diese Überzüge können gegebenenfalls auch stabilisierende, die Haftung verbessernde oder farbgebende, oder den Schmelzpunkt erniedrigende Zusätze enthalten.

Als für die Beschichtung geeignete Substrate seien beispielsweise genannt; Glas, Metalle, Metalloxide, wie Aluminiumoxid, ferner organische Polymere.

Das Beschichten der Substrate kann in der Weise erfolgen, daß man die Substrate auf eine Temperatur oberhalb des Schmelzpunktes der N-Phenyl-pyridinium-TCNQ-Komplexe erhitzt und anschließend die festen TCNQ-Komplexe auf diese heißen Substratoberflächen aufstreut. Dieses Aufschmelzen der TCNQ-Komplexe auf die erhitzten Substratoberflächen kann gegebenenfalls in einer Schutzgasatmosphäre, z.B. unter Wasserstoff, Stickstoff, Argon oder Helium oder aber auch im Vakuum vorgenommen werden.

Bei einem anderen Verfahren geht man so vor, daß man die TCNQ-Komplexe der Formel (II) bei Raumtemperatur auf die zu beschichtenden Substrate aufbringt und anschließend in einem vorgeheizten Ofen aufschmilzt.

Ein drittes Verfahren zur Aufbringung besteht darin, die Substrate durch Tauchen in eine Schmelze der TCNQ-Komplexe der Formel (II) zu beschichten.

Alle diese Verfahren führen zu elektrisch leitfähigen Überzugen auf den Substraten. Die so hergestellten, mit einem elektrisch leitfähigen Überzug versehenen Substrate finden Verwendung in der Elektronikindustrie.

Die erfindungsgemäßen N-Phenyl-pyridinium-TCNQ-Komplexe eignen sich auch für eine Einarbeitung in Kunststoffe, z.B. in Polycarbonat, um diese antistatisch auszurüsten.

Beispiel 1

Eine Suspension von 8,2 g (0,04 Mol) TCNQ in 200 ml Acetonitril wird bei Rückflußtemperatur unter Rühren mit einer Lösung von 8,5 g (0,03 Mol) N-Phenylpyridiniumiodid in 50 ml Acetonitril versetzt. Die Mischung wird anschließend noch 10 Minuten bei Rückflußtemperatur gerührt.

Beim Abkühlen scheidet sich aus der Lösung ein kristalliner Niederschlag ab. Dieser wird nach 12-stündigem Stehen abgesaugt, mit wenig Acetonitril gewaschen und getrocknet.

Ausbeute: 7,9 g (70 % der Theorie) des N-Phenyl-pyridinium-TCNQ-Komplexes der Formel

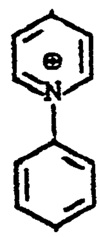

$(TCNQ^{\ominus\bullet})(TCNQ)$

Bestimmung des Schmelzverhaltens und der thermischen Stabilität verschiedener TCNQ-Komplexe und des vorstehend beschriebenen N-Phenyl-pyridinium-TCNQ-Komplexes:

Jeweils 0,5 g TCNQ-Komplex werden 1 Minute bei der in der nachstehenden Tabelle angegebenen Temperatur T geschmolzen. Es werden die Leitfähigkeiten der verschiedenen TCNQ-Komplexe vor dem Schmelzen ($\sigma^{o}$) und der wiedererstarrten Schmelzen ($\sigma^{o}$) bestimmt (Vierelektrodenmessung unter Druck 250 $Kp/cm^2$).

In der nachstehenden Tabelle sind die verschiedenen untersuchten TCNQ-Komplexe, die zum Schmelzen erforderlichen Temperaturen und die erhaltenen $\sigma^{o}$ und $\sigma^{s}$-Werte zusammengestellt.

Tabelle

| $K^{\oplus}$ | T (°C) | $\sigma^{o}$ (S/cm) | $\sigma^{s}$ (S/cm) |
|---|---|---|---|
| 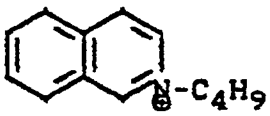 (gemäß DE-OS 32 14 355) | 250 | 0,1 | 0,08 |
| 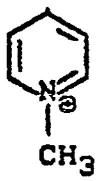 (gemäß DE-OS 23 29 492) | 280 | $5 \times 10^{-3}$ | $10^{-7}$ |
| 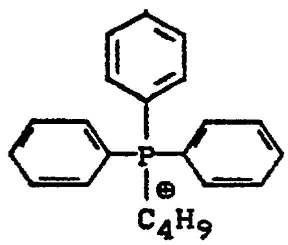 (gemäß DE-OS 34 17 466) | 230 | $7 \times 10^{-3}$ | $4 \times 10^{-3}$ |
| 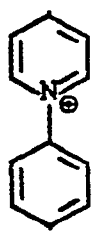 (gemäß Beispiel 1) | 280 | 0,5 | 0,3 |

Beispiel 2

Die siedende Lösung von 1,42 g (6,7 mmol) $Li^{\oplus}(TCNQ^{\ominus})$ in 20 ml wäßrigem Methanol ($CH_3OH:H_2O$ = 10:1) wird mit der Lösung von 2,0 g (6,8 mmol) N-p-Tolylpyridiniumiodid in 13 ml wäßrigem Methanol ($CH_3OH:H_2O$ = 10:1) versetzt. Die Mischung wird 10 Minuten auf Rückflußtemperatur erhitzt.

Nach dem Abkühlen wird der Niederschlag abgesaugt, mit wenig kaltem Wasser gewaschen und getrocknet.

Ausbeute: 2,14 g (85 % der Theorie) des N-p-Tolylpyridinium-TCNQ-Komplexes der Formel

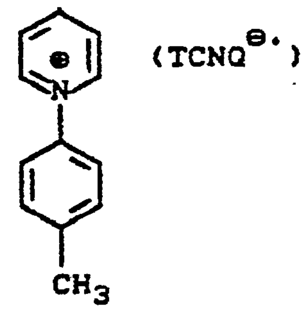

Die 2,14 g (5,7 mmol) N-p-Tolylpyridinium-TCNQ-Salz werden mit 1,16 g (5,7 mmol) TCNQ in 100 ml Acetonitril 10 Minuten auf Rückflußtemperatur erhitzt. Nach dem Abkühlen wird der kristalline Niederschlag abgesaugt, mit wenig kaltem Acetonitril gewaschen und getrocknet.

Ausbeute: 2,89 g (87 % der Theorie) des N-p-Tolyl-pyridinium-TCNQ-Komplexes der Formel

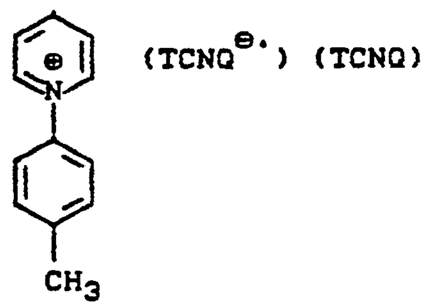

**Patentansprüche**

1. N-Arylpyridinium-7,7,8,8-tetracyano-p-chinodimethan-(TCNQ)-Komplexe der Formel

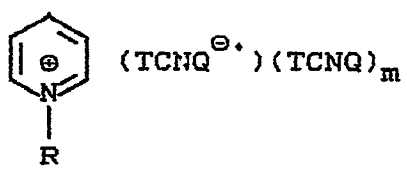 (II)

in der
m Null oder eine ganze oder gebrochene Zahl von 1 bis 2 ist und
R für einen gegebenenfalls durch $C_1$–$C_4$-Alkylreste, $C_1$–$C_4$-Alkoxyreste, Halogenatome, $C_1$–$C_5$-Acylreste substituierten Phenylrest steht.

2. Verwendung der Komplexe nach Anspruch 1 zur Herstellung elektrisch leitfähiger Überzüge durch Aufschmelzen auf Substraten.

3. Verwendung der Komplexe nach Anspruch 1 zur Herstellung elektrisch leitfähiger Überzüge durch Tauchbeschichtung mit den TCNQ-Komplexschmelzen.

**Revendications**

1. Complexes de N-arylpyridinium de 7,7,8,8-tétracyano-p-quinodiméthane (TCNQ) de formule

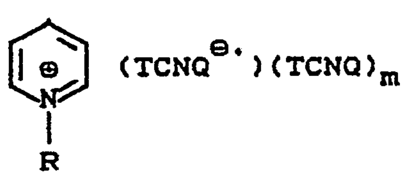 (II)

dans laquelle

m est égal à zéro ou à un nombre entier ou fractionnaire ayant une valeur de 0 à 2 et
R représente un reste phényle substitué le cas échéant par des restes alkyle en $C_1$ à $C_4$, des restes alkoxy en $C_1$ à $C_4$, des atomes d'halogènes, des restes acyle en $C_1$ à $C_5$.

2. Utilisation des complexes suivant la revendication 1 pour la production de revêtements conduisant l'éléctricité par application à l'état fondu sur des substrats.

3. Utilisation des complexes suivant la revendication 1 pour la production de revêtements conduisant l'éléctricité par immersion avec les masses fondues de complexes de TCNQ.

**Claims**

1. N-arylpyridinium-7,7,8,8-tetracyano-p-quinodimethyne (TCNQ) complexes of the formula

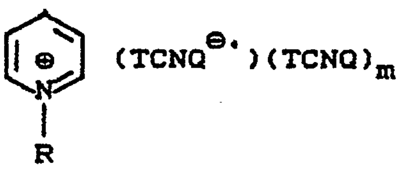

(II)

in which
m is zero or an integer or a fractional number from 0 to 2, and
R represents a pheynl radical optionally substituted by $C_1$–$C_4$-alkyl radicals, $C_1$–$C_4$-alkoxy radicals, halogenatoms or $C_1$–$C_5$-acyl radicals.

2. Use of the complexes according to Claim 1 for preparing electrically conductive coating produced by melting-on on substrates.

3. Use of the complexes according to Claim 1 for preparing electrically conductive coating produces by dipcoating with the TCNQ complex melts.